(19) Europäisches Patentamt
European Patent Office
Office européen des brevets

(11) EP 0 837 851 B1

(12) EUROPÄISCHE PATENTSCHRIFT

(45) Veröffentlichungstag und Bekanntmachung des Hinweises auf die Patenterteilung:
**29.05.2002 Patentblatt 2002/22**

(21) Anmeldenummer: **96922845.1**

(22) Anmeldetag: **19.06.1996**

(51) Int Cl.[7]: **C07D 221/10**, C09K 19/34, C09K 19/32, C07C 23/44, C07C 43/225

(86) Internationale Anmeldenummer:
**PCT/EP96/02653**

(87) Internationale Veröffentlichungsnummer:
**WO 97/02247 (23.01.1997 Gazette 1997/05)**

(54) **FLUORIERTE PHENANTHREN-DERIVATE UND IHRE VERWENDUNG IN FLÜSSIGKRISTALLINEN MISCHUNGEN**

FLUORINATED PHENANTHRENE DERIVATIVES AND THEIR USE IN LIQUID CRYSTAL MIXTURES

DERIVES DE PHENANTHRENE FLUORES ET LEUR UTILISATION DANS DES MELANGES A CRISTAUX LIQUIDES

(84) Benannte Vertragsstaaten:
**DE FR GB**

(30) Priorität: **04.07.1995 DE 19524230**

(43) Veröffentlichungstag der Anmeldung:
**29.04.1998 Patentblatt 1998/18**

(73) Patentinhaber: **Aventis Research & Technologies GmbH & Co. KG**
**65926 Frankfurt am Main (DE)**

(72) Erfinder:
- **MANERO, Javier**
  **D-65931 Frankfurt am Main (DE)**
- **WINGEN, Rainer**
  **D-65795 Hattersheim (DE)**

(74) Vertreter: **Bardehle, Heinz, Dipl.-Ing. et al**
**Patent- und Rechtsanwälte**
**Bardehle . Pagenberg . Dost . Altenburg . Geissler . Isenbruck**
**Theodor-Heuss-Anlage 12**
**68165 Mannheim (DE)**

(56) Entgegenhaltungen:

| | |
|---|---|
| **WO-A-87/01717** | **DE-A- 4 402 986** |
| **DE-A- 19 500 768** | **FR-A- 2 243 925** |
| **FR-A- 2 490 233** | |

Bemerkungen:
Die Akte enthält technische Angaben, die nach dem Eingang der Anmeldung eingereicht wurden und die nicht in dieser Patentschrift enthalten sind.

EP 0 837 851 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Neben nematischen und cholesterischen Flüssigkristallen werden in jüngerer Zeit auch optisch aktive geneigt smektische (ferroelektrische) Flüssigkristalle in kommerziellen Displayvorrichtungen verwendet.

Clark und Lagerwall konnten zeigen, daß der Einsatz ferroelektrischer Flüssigkristalle (FLC) in sehr dünnen Zellen zu optoelektrischen Schalt- oder Anzeigeelementen führt, die im Vergleich zu den herkömmlichen TN ("twisted nematic")-Zellen um bis zu einem Faktor 1000 schnellere Schaltzeiten haben (siehe z.B. EP-A 0 032 362). Aufgrund dieser und anderer günstiger Eigenschaften, z.B. der bistabilen Schaltmöglichkeit und des nahezu blickwinkelunabhängigen Kontrasts, sind FLCs grundsätzlich für Anwendungsgebiete wie Computerdisplays gut geeignet.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S_A$ und $S^*_c$-Phase läßt sich z.B. erreichen, wenn die Phasenfolge der Flüssigkristallmischung mit abnehmender Temperatur lautet:

$$\text{Isotrop} \rightarrow N^* \rightarrow S_A \rightarrow S^*_c$$

Vorraussetzung ist, daß der Pitch (Ganghöhe der Helix) in der N*-Phase sehr groß (größer 10 μm) oder, noch besser, völlig kompensiert ist (siehe z.B. T. Matsumoto et al., p. 468-470, Proc. of the 6th Int. Display Research Conf., Japan Display, Sept. 30 - Okto. 2, 1986, Tokyo, Japan; M. Murakami et al., ibid. S. 344 - S. 347). Dies erreicht man z. B., indem man zu der chiralen Flüssigkristallmischung, die in der N*-Phase z.B. eine linksdrehende Helix aufweist, einen oder mehrere optisch aktive Dotierstoffe, die eine rechtsdrehende Helix induzieren, in solchen Mengen hinzugibt, daß die Helix kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Voraussetzung, daß der Pitch in der smektischen C*-Phase wesentlich größer ist als die Dicke des Anzeigeelementes (Mol. Cryst. Liq. Cryst. **1983,** 94, 213 und **1984,** 114, 151. Dies erreicht man, wie im Fall des cholesterischen Pitches, durch Verwendung von Dotierstoffen mit entgegengesetztem Drehsinn der Helix.

Die optische Schaltzeit $\tau$ [μs] ferroelektrischer Flüssigkristallsysteme, die möglichst kurz sein soll, hängt von der Rotationsviskosität des Systems $\gamma$ [mPas], der spontanen Polarisation $P_s$[nC/cm$^2$] und der elektrischen Feldstärke E[V/m] ab nach der Beziehung

$$\tau \sim \frac{\gamma}{P_s \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$, vorzugsweise $\approx 0{,}13$, und eine geringe positive oder vorzugsweise negative dielektrische Anisotropie $\Delta\in$ verlangt (siehe z.B. S.T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktserniedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Ferroelektrische Flüssigkristallanzeigen lassen sich auch durch Nutzung des DHF (Distorted Helix Formation) -Effektes oder des PSFLCD-Effektes (Pitch Stabilized Ferroelectric Liquid Crystal Display, auch SBF = Short Pitch Bistable Ferroelektric Effect genannt) betreiben. Der DHF-Effekt wurde von B.I. Ostrovski in Advances in Liquid Crystal Research and Applications, Oxford/Budapest 1980, 469. beschrieben, der PSFLCD-Effekt ist in DE-A 39 20 625 bzw. EP-A 0 405 346 beschrieben. Zur Nutzung dieser Effekte wird im Gegensatz zum SSFLCD-Effekt ein flüssigkristallines

Material mit einem kurzen $S_C$-Pitch benötigt.

Derivate des Phenanthrens (wozu hier auch 9,10-Dihydrophenanthrene gezählt werden) wurden bereits als Flüssigkristalle bzw. als Komponenten flüssigkristalliner Mischungen beschrieben:

Azomethine mit einer Phenanthren- bzw. 9,10-Dihydrophenanthren-Einheit (J. Chem. Soc. [London] **1958**, 552; J. Chem. Soc., Perkin II **1982,** 465); Keto-Derivate des 9,10-Dihydrophenanthrens bzw. Phenanthrens (Chem. Ind. [London] **1974**, 615; Prod. Int. Liq. Cryst. Conf. **1973**, 397; Tetrahedron **1981**, 37, 2815; Carboxyl-Derivate des 9,10-Dihydrophenanthrens (DD-WP 153 826); 2,7-Bis(alkyloxy)phenanthrene (Nippon Kagaku Kaishi **1980**, 250).

Beispiele für die Verwendung von Pherianthrenderivaten in ferroelektrischen Flüssigkristallmischungen sind aus der WO 87/01717 bekannt. Die Verwendung von Dihydrophenanthrenderivaten ist aus der FR 7333122 und der FR 2490 233 bekannt. Aus der DE 44 02 986 A1 ist Verwendung von Phenanthridinderivaten in flüssigkristallinen Mischungen bekannt.

Da aber die Entwicklung, insbesondere von ferroelektrischen Flüssigkristallmischungen, noch in keiner Weise als abgeschlossen betrachtet werden kann, sind die Hersteller von Displays an den unterschiedlichsten Komponenten für Mischungen interessiert. Dieses u.a. auch deshalb, weil erst das Zusammenwirken der flüssigkristallinen Mischungen mit den einzelnen Bauteilen der Anzeigevorrichtung bzw. der Zellen (z.B. der Orientierungsschicht) Rückschlüsse auf die Qualität auch der flüssigkristallinen Mischungen zuläßt.

Aufgabe der vorliegenden Erfindung war es daher, neue Verbindungen bereitzustellen, die in flüssigkristallinen Mischungen geeignet sind, das Eigenschaftsprofil dieser Mischungen zu verbessern.

Es wurde nun gefunden, daß 2,7-disubstituierte Phenanthren-Derivate der Formel (I) in besonderer Weise zum Einsatz in Flüssigkristallmischungen geeignet sind.

Gegenstand der Erfindung sind daher Verbindungen der Formel (I),

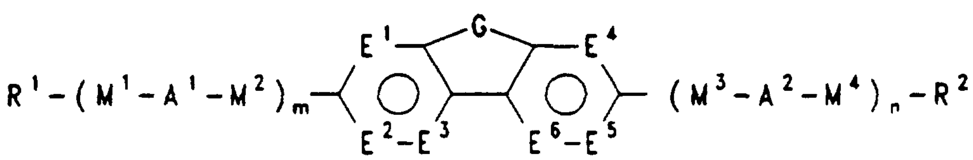

(I)

in der die Symbole und Indizes folgende Bedeutungen haben: $E^1$, $E^2$, $E^3$, $E^4$, $E^5$, $E^6$ sind gleich oder verschieden -N-, -CF- oder -CH-.

Bevorzugt sind Verbindungen der Formel (I) bei denen $E^1$, $E^2$, $E^3$, $E^4$, $E^5$, $E^6$ gleich oder verschieden -CF- oder -CH- sind.

Besonders bevorzugt sind Verbindungen der Formel (I), in denen $E^1$, $E^2$, $E^3$, $E^4$, $E^5$, $E^6$ -CH- sind oder in denen $E^2$, $E^3$, $E^5$, $E^6$ -CH- und $E^1$, $E^4$ -CF- sind.

G ist $-CF_2CF_2-$ oder -CF = CF-,

$R^1$, $R^2$ sind gleich oder verschieden Wasserstoff, -CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 20 C-Atomen (mit oder ohne asymmetrisches C-Atom), wobei auch eine oder mehrere $CH_2$- Gruppen durch -O-, -S-, -CO-O-, -O-CO-, -O-CO-O-, -CO-, -CS-, -CH=CH-, -C≡C-, Cyclopropan-1,2-diyl, $-Si(CH_3)_2-$, 1,4-Phenylen, trans-1,4-Cyclohexylen oder trans-1,3-Cyclopentylen ersetzt sein können, mit der Maßgabe, daß Sauerstoffatome und/oder Schwefelatome nicht unmittelbar miteinander gebunden sein dürfen, und/oder wobei ein oder mehrere H-Atome des Alkylrestes durch -F, -Cl, -Br, $-OR^3$, -SCN, -OCN oder $-N_3$ substituiert sein können, oder auch eine der nachfolgenden Gruppen (optisch aktiv oder racemisch):

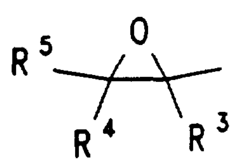

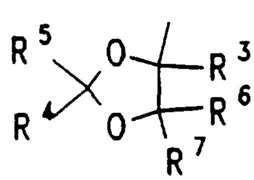

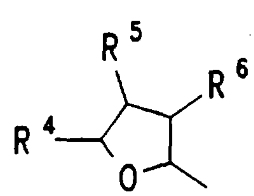

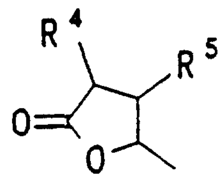 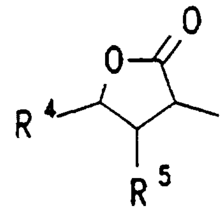 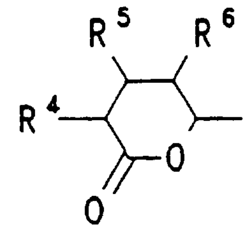

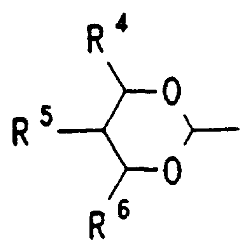 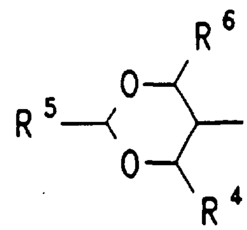 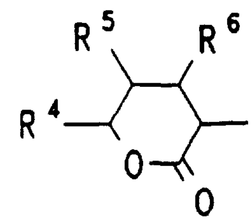

6

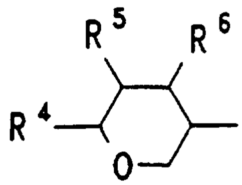 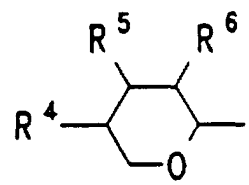 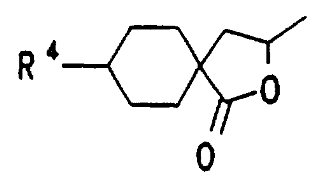

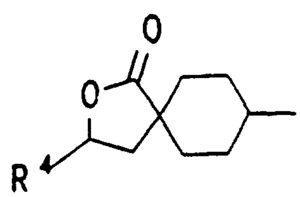 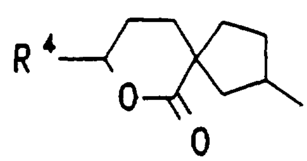 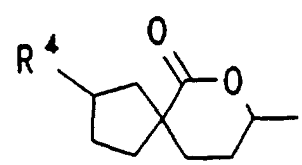

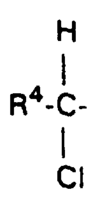 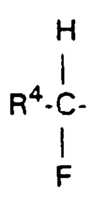 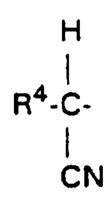 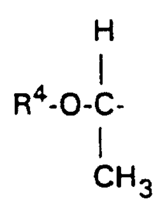 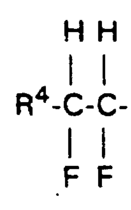

Bevorzugt sind $R^1$, $R^2$ gleich oder verschieden ein geradkettiger oder verzweigter Alkylrest (mit oder ohne asymmetrisches C-Atom) mit 1 bis 16 C-Atomen, wobei auch eine oder mehrere $CH_2$-Gruppen durch -O-, Cyclopropan-1,2-diyl oder $-Si(CH_3)_2-$ ersetzt sein können, mit der Maßgabe, daß Sauerstoffe nicht unmittelbar verbunden sein dürfen, und/oder wobei ein oder mehrere H-Atome des Alkylrestes durch F substituiert sein können; $R^1$ oder $R^2$ können auch H sein, jedoch nicht beide gleichzeitig.

Besonders bevorzugt sind $R^1$, $R^2$ gleich oder verschieden geradkettige oder verzweigte Alkyl- oder Alkyloxy-Reste mit 1 bis 10 C-Atomen sind, wobei auch eine, durch mindestens zwei weitere $CH_2$-Gruppen vom Kern getrennte, $CH_2$-Gruppe durch $-Si(CH_3)_2-$ ersetzt sein kann.

$R^3$, $R^4$, $R^5$, $R^6$, $R^7$ sind gleich oder verschieden Wasserstoff oder ein geradkettiger oder verzweigter Alkylrest mit 1-16 C-Atomen (mit oder ohne asymmetrisches C-Atom), wobei auch eine oder mehrere $CH_2$-Gruppen durch -O- und/oder -CH =CH- ersetzt sein können, mit der Maßgabe, daß Sauerstoffatome nicht unmittelbar miteinander gebunden sein dürfen, und/oder wobei ein oder mehrere H-Atome des Alkylrestes durch -F oder -Cl substituiert sein können; $R^4$ und $R^5$ können zusammen auch $-(CH_2)_4-$ oder $-(CH_2)_5-$ sein, wenn sie an ein Oxiran-, Dioxolan-, Tetrahydrofuran-, Tetrahydropyran-, Butyrolacton- oder Valerolacton-System gebunden sind.

$M^1$, $M^2$, $M^3$, $M^4$ sind gleich oder verschieden -O-, -S-, -CO-, -CO-O-, -O-CO-, -O-CO-O-, -CO-S-, -S-CO-, -CS-O-, -O-CS-, -S-CS-S-, -O-CS-O, -S-CO-S-, -CS-, $-CH_2-O-$, $-O-CH_2-$, $-CH_2-S-$, $-S-CH_2-$, -CH=CH-, -C≡C-, $-CH_2-CH_2-CO-O-$, $-O-CO-CH_2-CH_2-$ oder eine Einfachbindung.

Bevorzugt sind $M^1$, $M^2$, $M^3$, $M^4$ gleich oder verschieden -O-, -CO-O-, -O-CO-, -O-CO-O-, $-CH_2-O-$, $-O-CH_2-$, -CH=CH-, -C≡C-, $-CH_2-CH_2-CO-O-$, $-O-CO-CH_2-CH_2-$ oder eine Einfachbindung.

Besonders bevorzugt sind $M^1$, $M^2$, $M^3$, $M^4$ gleich oder verschieden -O-, -CO-O-, -O-CO-, $-CH_2-O-$, $-O-CH_2-$ oder eine Einfachbindung.

$A^1$, $A^2$ sind gleich oder verschieden 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, wobei ein oder zwei H-Atome durch CN und/oder $CH_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F, Cl und/oder CN ersetzt sein kann, 1,3-Thiazol-2,5-diyl, wobei ein H-Atom durch F, Cl und/oder CN ersetzt sein kann, Thiophen-2,4-diyl, wobei ein H-Atom durch F, Cl und/oder CN ersetzt sein kann, Thiophen-2,5-diyl, wobei ein oder zwei H-Atome durch F, Cl und/oder CN ersetzt sein können, Piperazin-1,4-diyl, Piperazin-2,5-diyl, Naphthalin-2,6-diyl, wobei ein oder mehrere H-Atome durch F, Cl und/oder CN ersetzt sein können, Bicyclo[2.2.2]octan-1,4-diyl, wobei ein oder mehrere H-Atome durch F, Cl und/oder CN ersetzt sein können oder 1,3-Dioxaborinan-2,5-diyl.

Bevorzugt sind $A^1$, $A^2$ gleich oder verschieden 1,4-Phenylen, wobei ein oder mehrere H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, wobei ein oder zwei H-Atome durch CN und/oder $CH_3$ ersetzt sein können, (1,3,4)-Thiadiazol-2,5-diyl, 1,3-Dioxan-2,5-diyl, 1,3-Thiazol-2,4-diyl, wobei ein H-Atom durch F, Cl und/oder CN ersetzt sein kann, 1,3-Thiazol-2,5-diyl, wobei ein H-Atom durch F, Cl und/oder CN ersetzt sein kann, Thiophen-2,5-diyl, wobei ein oder zwei H-Atome durch F, Cl und/oder CN ersetzt sein können, oder Naphthalin-2,6-diyl, wobei ein oder mehrere H-Atome durch F, Cl und/oder CN ersetzt sein können.

Besonders bevorzugt sind $A^1$, $A^2$ gleich oder verschieden 1,4-Phenylen, wobei ein oder mehrere H-Atome durch F ersetzt sein können, Pyridin-2,5-diyl, wobei ein H-Atom durch F ersetzt sein kann, Pyrimidin-2,5-diyl, wobei ein H-Atom durch F ersetzt sein kann, trans-1,4-Cyclohexylen, wobei ein oder zwei H-Atome durch CN und/oder $CH_3$ ersetzt sein können, oder Naphthalin-2,6-diyl, wobei ein oder mehrere H-Atome durch F ersetzt sein können.

n, m sind Null oder Eins, jedoch in der Summe maximal 1.

Ganz besonders bevorzugt sind Verbindungen der Formel (Ia), in denen $E^1$ und $E^4$ gleich oder verschieden -CH- und/oder -CF- und $E^2$, $E^3$, $E^5$ und $E^6$ -CH-bedeuten:

(Ia) 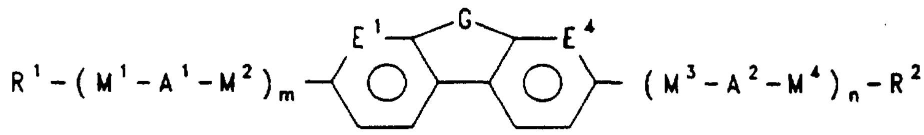

und darunter insbesondere die Verbindungen

(Ia1) 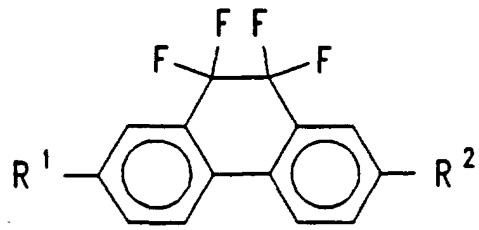

(Ia2) 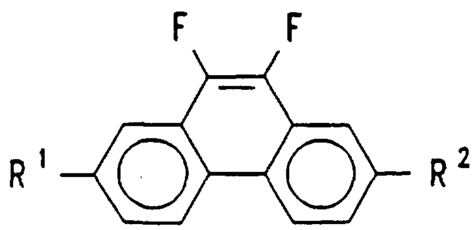

(Ia3) 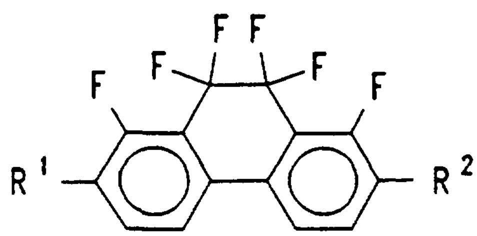

(Ia4) 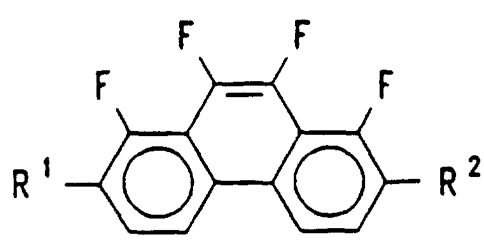

(Ia5) 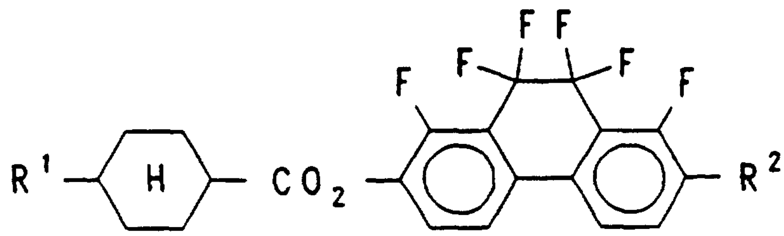

(Ia6) 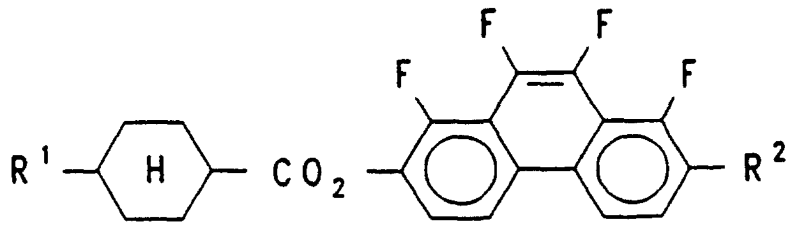

(Ia7) 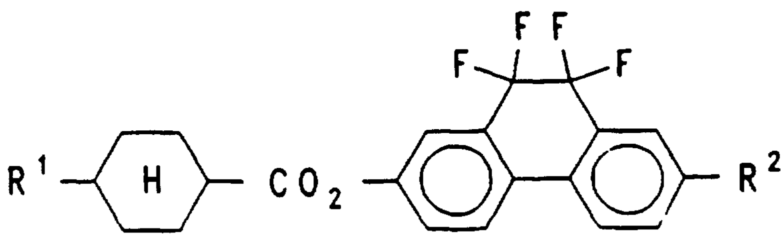

(Ia8) 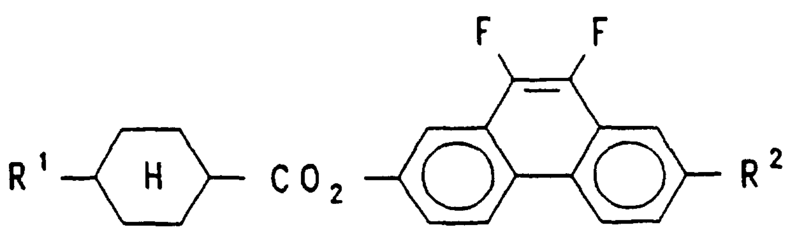

wobei $R^1$ und $R^2$ die in der Formel (I) angegebenen Bedeutungen haben.

Die Herstellung der erfindungsgemäßen Verbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch machen.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

Beispielhaft sind in den Schemata 1 und 2 Synthesewege zu Verbindungen der Formel Ia1 und Ia7 bzw. Ia2 und Ia8 angegeben, wobei auch andere Verfahren denkbar und möglich sind.

**Schema 1:**

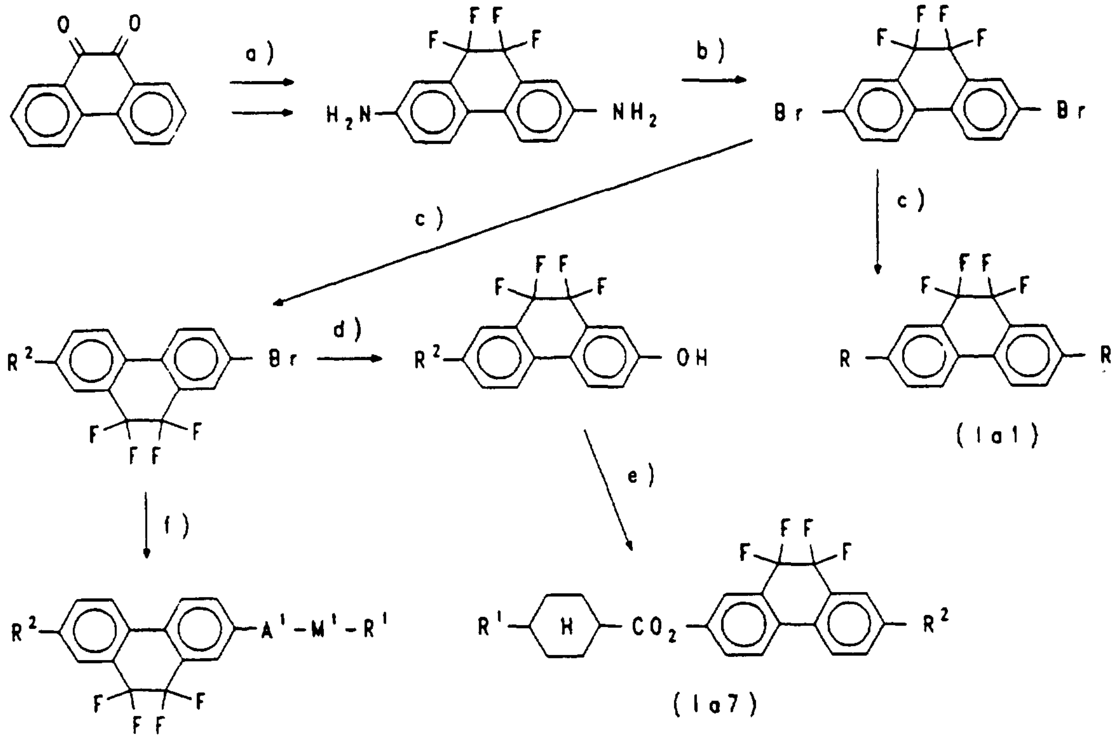


(Ia1)

(Ia7)

a) Zh. Obshch. Khim **1966**, 36, 1815;
b) 1) $NaNO_2$, 2) CuBr; (Sandmeyer-Reaktion);
c) $R^2CuLi$, Übersicht siehe Org. Reak. **1975**, 22, 253;
d) Glykol/KOH/S; analog DE-A 42 36 102;
e) HBTU, analog DE-A 44 27 198;
f) analog EP-A 0 694 530

## Schema 2:

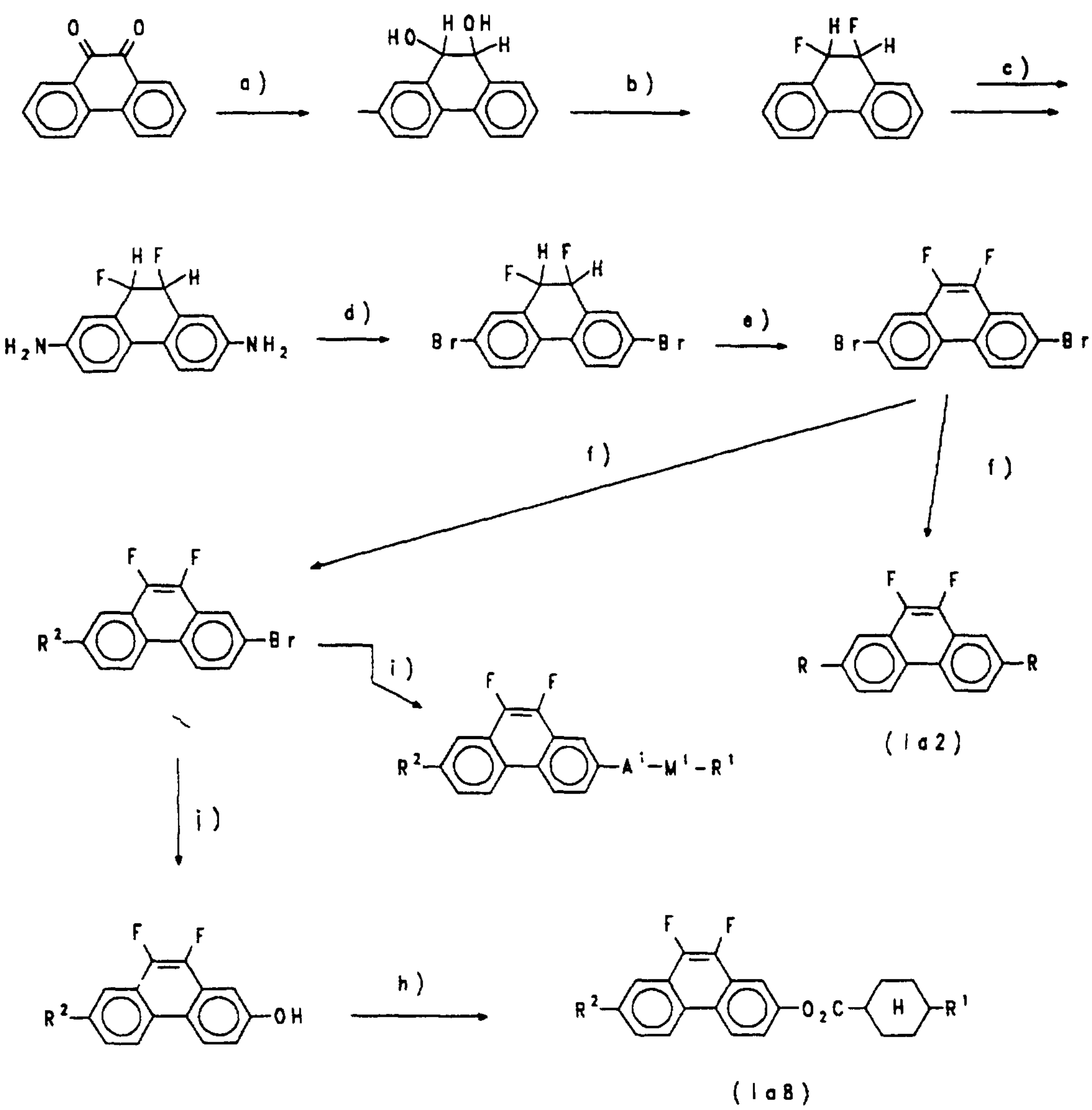


a) $LiAlH_4$; analog JACS **98** (1976) 8114.
b) z.B. Diethylaminoschwefeltrifluorid (DAST); analog J.Org. Chem. **40** (1975) 574.
c) analog Zh.Obshch. Khim **1966**, 36,1815;
d) 1. $NaNO_2$ 2. CuBr (Sandmeyer-Reaktion);
e) z.B. mit 2,3-Dichlor-5,6 dicyanobenzochinon; analog J.Chem. Soc. **1954,** 3569.
f) $R^2CuLi$, Übersicht siehe Org. Reak. **1975**, 22, 253;
g) Glykol, Kolt S, analog DE-A-42 36 102;
h) HBTU, analog DE-A-44 27 198;
i) analog EP-A-0 694 530

Die Synthese des Restes $R^1(-M^1-A^1-M^2)$ bzw. $(-M^3-A^2-M^4)-R^2$ erfolgt nach an sich bekannten, dem Fachmann geläufigen Methoden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch machen.

Beispielsweise sei verwiesen auf DE-A 23 44 732, 24 50 088, 24 29 093, 25 02 94, 26 36 684, 27 01 591 und 27 52 975 für Verbindungen mit 1,4-Cyclohexylen und 1,4-Phenylen-Gruppen; DE-A 26 41 724 für Verbindungen mit Pyrimidin-2,5-diyl-Gruppen; DE-A 40 26 223 und EP-A 03 91 203 für Verbindungen mit Pyridin-2,5-diyl-Gruppen; DE-A 32 31 462 für Verbindungen mit Pyridazin-3,6-diyl-Gruppen; EP-A 309 514 für Verbindungen mit (1,3,4)-Thiadiazol-2-5-diyl-Gruppen; WO-A 92/16500 für Naphthalin-2,6-diyl-Gruppen; DE-A 37 10 890 für Bicyclo[2.2.2.]octan-1,4-diyl-Gruppen; K. Seto et al, Journal of the Chemical Society, Chemical Communications 1988, 56 für Dioxborinan-2,5-diyl-Gruppen.

Die Herstellung disubstituierter Pyridine, disubstituierter Pyrazine, disubstituierter Pyrimidine und disubstituierter Pyridazine findet sich beispielsweise auch in den entsprechenden Bänden der Serie "The Chemistry of Heterocyclic Compounds" von A. Weissberger und E.C. Taylor (Herausgeber).

Dioxanderivate werden zweckmäßig durch Reaktion eines entsprechenden Aldehyds (oder eines seiner reaktionsfähigen Derivate) mit einem entsprechenden 1,3-Diol (oder einem seiner reaktionsfähigen Derivate) hergestellt, vorzugsweise in Gegenwart eines inerten Lösungsmittels, wie Benzol oder Toluol, und/oder eines Katalysators, z.B. einer starken Säure, wie Schwefelsäure, Benzol- oder p-Toluolsulfonsäure, bei Temperaturen zwischen etwa 20°C und etwa 150°C, vorzugsweise zwischen 80°C und 120°C. Als reaktionsfähige Derivate der Ausgangsstoffe eignen sich in erster Linie Acetale.

Die genannten Aldehyde und 1,3-Diole sowie ihre reaktionsfähigen Derivate sind zum Teil bekannt, zum Teil können sie ohne Schwierigkeiten nach Standardverfahren der Organischen Chemie aus literaturbekannten Verbindungen hergestellt werden. Beispielsweise sind die Aldehyde durch Oxydation entsprechender Alkohole oder durch Reduktion von Nitrilen oder entsprechenden Carbonsäuren oder ihrer Derivate, die Diole durch Reduktion entsprechender Diester erhältlich.

Verbindungen, worin ein aromatischer Ring durch mindestens ein F-Atom substituiert ist, können auch aus den entsprechenden Diazoniumsalzen durch Austausch der Diazoniumgruppe gegen ein Fluoratom, z.B. nach den Methoden von Balz und Schiemann, erhalten werden.

Was die Verknüpfung der Ringsysteme miteinander angeht, sei beispielsweise verwiesen auf:
N. Miyaura, T. Yanagai und A. Suzuki in Synthetic Communications 11 (1981), 513-519 DE-C-39 30 663, M.J. Sharp, W. Cheng, V. Snieckus in Tetrahedron Letters 28 (1987) 5093; G.W. Gray in J. Chem. Soc. Perkin Trans II 1989, 2041 und Mol. Cryst. Liq. Cryst. 172 (1989) 165, 204 (1991) 43 und 91; EP-A 0 449 015; WO-A 89/12039; WO-A 89/03821; EP-A O 354 434 und EP-A 0 694 530 für die direkte Verknüpfung von Aromaten und Heteroaromaten; DE-A 32 01 721 für Verbindungen mit $-CH_2CH_2-$Brückengliedern und Koji Seto et al. in Liquid Crystals 8 (1990) 861-870 für Verbindungen mit $-C{\equiv}C-$Brückengliedern.

Ester der Formel (I) können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt und können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls, wie Natrium oder Kalium, in Betracht.

Die Veresterung wird vorteilhaft in Gegenwart eines inerten Lösungsmittels durchgeführt. Gut geeignet sind insbesondere Ether, wie Diethylether, Di-n-butylether, THF, Dioxan oder Anisol, Ketone, wie Aceton, Butanon oder Cyclohexanon, Amide, wie DMF oder Phosphorsäurehexamethyltriamid, Kohlenwasserstoffe, wie Benzol, Toluol oder Xylol, Halogenkohlenwasserstoffe, wie Tetrachlorkohlenstoff, Dichlormethan oder Tetrachlorethylen und Sulfoxide, wie Dimethylsulfoxid oder Sulfolan.

Ether der Formel (I) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, Alkylsulfonat oder Dialkylsulfat umgesetzt werden, zweckmäßig in einem inerten Lösungsmittel, wie Aceton, 1,2-Dimethoxyethan, DMF oder Dimethylsulfoxid, oder auch mit einem Überschuß an wäßriger oder wäßrig-alkoholischer NaOH oder KOH bei Temperaturen zwischen etwa 20° und 100°C.

Was die Synthese spezieller Reste $R^1$ angeht, sei zusätzlich beispielsweise verwiesen auf EP-A 0 355 008 für Verbindungen mit siliziumhaltigen Seitenketten und EP-A 0 292 954 und EP-A 0 398 155 für Verbindungen mit Cyclopropylgruppen in der Seitenkette.

Mit der Bereitstellung von Verbindungen der Formel (I) wird ganz allgemein die Palette der flüssigkristallinen Substanzen, die sich unter verschiedenen anwendungstechnischen Gesichtspunkten zur Herstellung flüssigkristalliner

Gemische eignen, erheblich verbreitert.

In diesem Zusammenhang besitzen die Verbindungen der Formel (I) einen breiten Anwendungsbereich. In Abhängigkeit von der Auswahl der Substituenten können sie als Basismaterialien dienen, aus denen flüssigkristaliine Phasen zum überwiegenden Teil zusammengesetzt sind; es können aber auch Verbindungen der Formel (I) flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Besonders geeignet sind die Verbindungen der Formel (I), um schon in geringen Zumischmengen die dielektrische Anisotropie ($\Delta\varepsilon$) in Richtung auf höhere negative Werte zu beeinflussen.

Gegenstand der Erfindung ist auch die Verwendung von Verbindungen der Formel (l) in Flüssigkristallmischungen, vorzugsweise ferroelektrischen und nematischen, insbesondere ferroelektrischen.

Weiterhin Gegenstand der Erfindung sind Flüssigkristallmischungen, vorzugsweise ferroelektrische und nematische, insbesondere ferroelektrische, enthaltend eine oder mehrere Verbindungen der Formel (l).

Die erfindungsgemäßen Flüssigkristallmischungen enthalten im allgemeinen 2 bis 35, vorzugsweise 2 bis 25, besonders bevorzugt 2 bis 20 Komponenten.

Sie enthalten im allgemeinen 0,01 bis 80 Gew.-%, vorzugsweise 0,1 bis 60 Gew.-%, besonders bevorzugt 0,1 bis 30 Gew.-%, an einer oder mehreren, vorzugsweise 1 bis 10, besonders bevorzugt 1 bis 5, ganz besonders bevorzugt 1 bis 3, der erfindungsgemäßen Verbindungen der Formel (l).

Weitere Komponenten von Flüssigkristallmischungen, die erfindungsgemäße Verbindungen der Formel (I) enthalten, werden vorzugsweise ausgewählt aus den bekannten Verbindungen mit smektischen und/oder nematischen und/oder cholesterischen Phasen. Dazu gehören z. B.:

- Derivate des Phenylpyrimidins, wie beispielsweise in WO 86/06401, US-A 4 874 542 beschrieben,
- metasubstituierte Sechsringaromaten, wie beispielsweise in EP-A 0 578 054 beschrieben,
- Siliziumverbindungen, wie beispielsweise in EP-A 0 355 008 beschrieben,
- mesogene Verbindungen mit nur einer Seitenkette, wie beispielsweise in EP-A 0 541 081 beschrieben,
- Hydrochinonderivate, wie beispielsweise in EP-A 0 603 786 beschrieben,
- Pyridylpyrimidine, wie beispielsweise in W0 92/12974 beschrieben,
- Phenylbenzoate, wie beispielsweise bei P. Keller, Ferroelectrics **1984**, 58, 3 und J. W. Goodby et al., Liquid Crystals and Ordered Fluids, Bd. 4, New York 1984 beschrieben und
- Thiadiazole, wie beispielsweise in EP-A 0 309 514 beschrieben.

Als chirale, nicht racemische Dotierstoffe kommen beispielsweise in Frage:

- optisch aktive Phenylbenzoate, wie beispielsweise bei P. Keller, Ferroelectrics **1984**, 58, 3 und J. W. Goodby et al., Liquid Crystals and Ordered Fluids, Bd. 4, New York **1984** beschrieben,
- optisch aktive Oxiranether, wie beispielsweise in EP-A 0 263 437 und WO-A 93/13093 beschrieben,
- optisch aktive Oxiranester, wie beispielsweise in EP-A 0 292 954 beschrieben,
- optisch aktive Dioxolanether, wie beispielsweise in EP-A 0 351 746 beschrieben,
- optisch aktive Dioxolanester, wie beispielsweise in EP-A 0 361 272 beschrieben,
- optisch aktive Tetrahydrofuran-2-carbonsäureester, wie beispielsweise in EP-A 0 355 561 beschrieben, und
- optisch aktive 2-Fluoralkylether, wie beispielsweise in EP-A 0 237 007 und US-A 5,051,506 beschrieben.

Die Mischungen wiederum können Anwendung finden in elektrooptischen oder vollständig optischen Elementen, z. B. Anzeigeelementen, Schaltelementen, Lichtmodulatoren, Elementen zur Bildbearbeitung und/oder Signalverarbeitung oder allgemein im Bereich der nichtlinearen Optik.

Flüssigkristalline Mischungen, die Verbindungen der allgemeinen Formel (l) enthalten, sind besonders für die Verwendung in elektrooptischen Schalt- und Anzeigevorrichtungen (Displays) geeignet. Diese Displays sind üblicherweise so aufgebaut, daß eine Flüssigkristallschicht beiderseitig von Schichten eingeschlossen ist, die üblicherweise, in dieser Reihenfolge ausgehend von der LC-Schicht, mindestens eine Orientierungsschicht, Elektroden und eine Begrenzungsscheibe (z.B. aus Glas) sind. Darüberhinaus können sie Abstandshalter, Kleberahmen, Polarisatoren sowie für Farbdisplays dünne Farbfilterschichten enthalten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (siehe z.B. E. Kaneko, "Liquid Crystal TV Displays: Principles and Applications of Liquid Crystal Displays", KTK Scientific Publishers 1987).

Ferner sind die Mischungen für Feldbehandlung, d. h. zum Betrieb in der Quasi-Bookshelf-Geometrie (QBG) (siehe z.B. H. Rieger et al., SID 91 Digest (Anaheim) 1991, 396) geeignet.

Ebenso sind die erfindungsgemäßen Mischungen geeignet für die Verwendung in ferroelektrischen Flüssigkristallanzeigen, die auf Nutzung des DHF-Effekts oder des PSFLCD-Effekts (Pitch Stabilized Ferroelectric Liquid Crystal Display, auch SBF = Short Pitch Bistable Ferroelectric Effect genannt) beruhen.

Daneben können die Verbindungen der Formel (I) auch als Komponenten von antiferroelektrischen Flüssigkristallmischungen Verwendung finden.

Die Erfindung wird durch die Beispiele näher erläutert,.

Beispiel 1: 9,9,10,10-Tetrafluoro-2,7-dihexyl-9,10-dihydrophenanthren

4,1 g 9,9,10,10-Tetrafluoro-2,7-dibrom-9,10-dihydrophenanthren werden in 250 ml THF gelöst und auf 0°C gekühlt. Man fügt 120 mg 1,3-Bis(diphenylphosphin)propan-nickel(II)-chlorid zu und tropft bei 0°C 50 mmol Hexylmagnesiumbromid in 100 THF zu. Es wird noch 4 Stunden nachgerührt, anschließend weitere 18 Stunden bei 5°C. Man säuert mit 1n HCl an, sättigt die Wasserphase mit NaCl und trennt die Phasen. Die Wasserphase wird noch mehrmals mit tert.-Butylmethylether extrahiert. Das Rohprodukt wird an Kieselgel chromatographisch gereinigt. Man erhält 3,45 g 9,9,10,10-Tetrafluoro-2,7-dihexyl-9,10-dihydrophenanthren.

Beispiel 2: 9,10-Difluoro-2,7-dihexyl-phenanthren

3,8 g 9,10-Difluoro-2,7-dibrom-phenanthren werden in 250 ml THF gelöst und auf 0°C gekühlt. Man fügt 120 mg 1,3-Bis(diphenylphosphin)propan-nickel(II)-chlorid zu und tropft bei 0°C 50 mmol Hexylmagnesiumbromid in 100 THF zu. Es wird noch 4 Stunden nachgerührt, anschließend weitere 18 Stunden bei 5°C. Man säuert mit 1n HCl an, sättigt die Wasserphase mit NaCl und trennt die Phasen. Die Wasserphase wird mehrmals mit tert.-Butylmethylether extrahiert. Das Rohprodukt wird an Kieselgel chromatographisch gereinigt. Man erhält 2,78 g 9,10-Difluoro-2,7-dihexyl-phenanthren.

Analog Beispiele 1 und 2 lassen sich 9,10-Difluoro-2,7-dibrom-phenanthren und 9,9,10,10-Tetrafluoro-2,7-dibrom-9,10-dihydrophenanthren mit anderen Grignardreagentien zu symmetrisch substituierten Verbindungen der Formel (Ia1) und (Ia2) umsetzen.

Beispiel 3: 9,9,10,10-Tetrafluoro-2-brom-7-octyl-9,10-dihydrophenanthren

Analog Beispiel 1 aus 4,1 g 9,9,10,10-Tetrafluoro-2,7-dibrom-phenanthren und 10 mmol Octylmagnesiumbromid. Als Katalysator werden 0,2 mmol 1,3-Bis(diphenylphosphin)butan-nickel(II)-chlorid verwendet. Nach Chromatographie erhält man 2,4 g 9,9,10,10-Tetrafluoro-2-brom-7-octyl-9,10-dihydrophenanthren.

Beispiel 4: 9,10-Difluoro-2-brom-7-octyl-phenanthren

Analog Beispiel 2 aus 4,1 g 9,10-Difluoro-2,7-dibrom-phenanthren und 10 mmol Octylmagnesiumbromid. Als Katalysator werden 0,2 mmol 1,3-Bis(diphenylphosphin)butan-nickel(II)-chlorid verwendet. Nach Chromatographie erhält man 2,1 g 9,10-Difluoro-2-brom-7-octyl-phenanthren.

Analog Beispiel 3 und 4 lassen sich 9,10-Difluoro-2,7-dibrom-phenanthren und 9,9,10,10-Tetrafluoro-2,7-dibrom-9,10-dihydrophenanthren mit anderen Grignardreagentien zu monoalkylsubstituierten Verbindungen der Formel (Ia1) und (Ia2) umsetzen.

Beispiel 5: 9,9,10,10-Tetrafluoro-2-hexyl-7-octyl-9,10-dihydrophenanthren

Analog Beispiel 1 aus 9,9,10,10-Tetrafluoro-2-brom-7-octyl-9,10-dihydrophenanthren und Hexylmagnesiumbromid. Als Katalysator wird 1,3-Bis(diphenylphosphin)propan-nickel(II)-chloridverwendet. Nach Chromatographie erhält man 9,9,10,10-Tetrafluoro-2-hexyl-7-octyl-9,10-dihydrophenanthren.

Beispiel 6: 9,10-Difluoro-2-hexyl-7-octyl-phenanthren

Analog Beispiel 2 aus 9,10-Difluoro-2-brom-7-octyl-phenanthren und 10 mmol Hexylmagnesiumbromid. Als Katalysator werden 0,2 mmol 1,3-Bis(diphenylphosphin)propan-nickel(II)-chlorid verwendet. Nach Chromatographie erhält man 9,10-Difluoro-2-hexyl-7-octyl-phenanthren.

Analog Beispiel 5 und 6 lassen sich 9,10-Difluoro-2-alkyl-7-brom-phenanthrene und 9,9,10,10-Tetrafluoro-2-alkyl-7-brom-9,10-dihydrophenanthrene mit weiteren Grignardreagentien unsymmetrisch substituierten Verbindungen der Formel (Ia1) und (Ia2) umsetzen.

Beispiel 7: 2-(4-Decyloxy-phenyl)-9,9,10,10-tetrafluoro-7-butyl-9,10-dihydrophenanthren

Aus 9,9,10,10-Tetrafluoro-2-brom-7-butyl-dihydrophenanthren und 4-Decyloxyphenylboronsäure mittels palladiumkatalysierter Suzukikopplung (analog Acc. Chem. Res. **1982**, 15, 178). Das Rohprodukt wird säulenchromatographisch gereinigt.

Beispiel 8: 2-(4-Decyloxy-phenyl)-9,10-difluoro-7-butyl-phenanthren

Aus 9,10-Difluoro-2-brom-7-butyl-phenanthren und 4-Decyloxyphenylboronsäure mittels Palladiumkatlysierte Suzukikopplung (analog Acc. Chem. Res. **1982**, 15, 178). Das Rohprodukt wird säulenchromatographisch gereinigt.

Beispiel 9: 2-Butoxy-5-(9,9,10,10-tetrafluoro-7-hexyl-9,10-dihydrophenanthren-2-yl)-pyridin

Aus 9,9,10,10-Tetrafluoro-2-brom-7-hexyl-9,10-dihydrophenanthren und 2-Butoxypyridin-5-boronsäure analog Beispiel 7.

Beispiel 10: 2-Butoxy-5-(9,10-difluoro-7-hexyl-phenanthren-3-yl)-pyridin

Aus 9,10-Difluoro-2-brom-7-hexyl-phenanthren und 2-Butoxypyridin-5-boronsäure analog Beispiel 8.

Analog Beispiele 7, 8, 9 und 10 lassen sich 9,10-Difluoro-2-alkyl-7-brom-phenanthrene, 9,10-Difluoro-2-alkoxyl-7-brom-phenanthrene, 9,9,10,10-Tetrafluoro-2-alkyl-7-brom-9,10-dihydrophenanthrene und 9,9,10,10-Tetrafluoro-2-alkyloxy-7-brom-9,10-dihydrophenanthrene mit weiteren Aryl- und Heteroarylboronsäuren zu unsymmetrisch subsituierten Verbindungen umsetzen.

## Patentansprüche

**1.** Fluorierte Phenanthrenderivate der Formel (I),

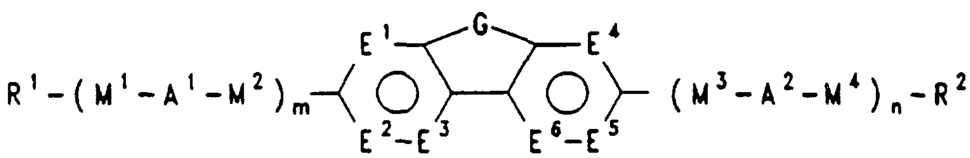

(I)

in der die Symbole und Indizes folgende Bedeutung haben:

| | |
|---|---|
| $E^1$, $E^2$, $E^3$, $E^4$, $E^5$, $E^6$ | gleich oder verschieden -N-, -CF- oder -CH-; |
| G | ist $-CF_2CF_2-$ oder -CF = CF-; |
| $R^1$, $R^2$ | sind gleich oder verschieden Wasserstoff, -CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 20 C-Atomen (mit oder ohne asymmetrisches C-Atom), wobei auch eine oder mehrere $CH_2$- Gruppen durch -O-, -S-, -CO-O-, -O-CO-, -O-CO-O-, -CO-, -CS-, -CH=CH-, -C≡C-, Cyclopropan-1,2-diyl, $-Si(CH_3)_2-$, 1,4-Phenylen, trans-1,4-Cyclohexylen oder trans-1,3-Cyclopentylen ersetzt sein können, mit der Maßgabe, daß Sauerstoffatome und/oder Schwefelatome nicht unmittelbar miteinander gebunden sein dürfen, und/oder wobei ein oder mehrere H-Atome des Alkylrestes durch -F, -Cl, -Br, $-OR^3$, -SCN, -OCN oder $-N_3$ substituiert sein können, oder auch eine der nachfolgenden Gruppen (optisch aktiv oder racemisch): |

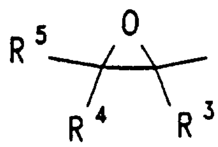
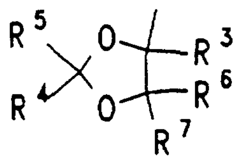
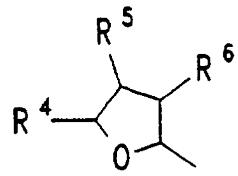

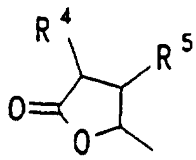
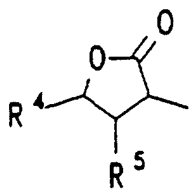
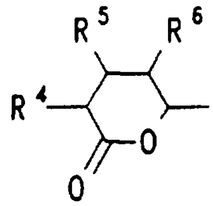

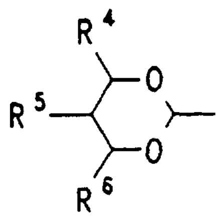
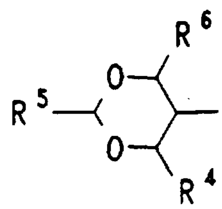
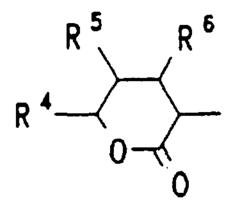

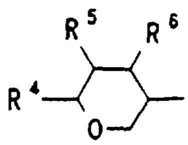
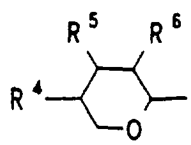
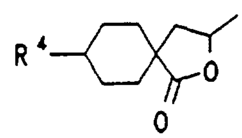

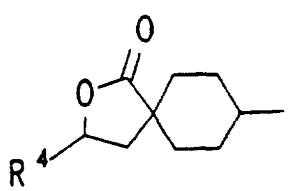
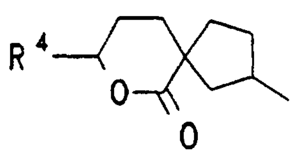
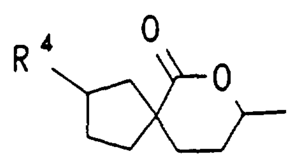

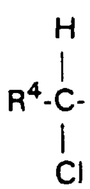
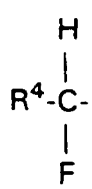
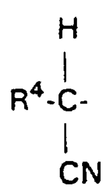
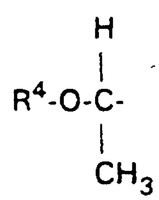
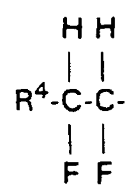

$R^3$, $R^4$, $R^5$, $R^6$, $R^7$ sind gleich oder verschieden Wasserstoff oder ein geradkettiger oder verzweigter Alkylrest mit 1-16 C-Atomen (mit oder ohne asymmetrisches C-Atom), wobei auch eine oder mehrere $CH_2$-Gruppen durch -O- und/oder -CH =CH- ersetzt sein können, mit der Maßgabe, daß Sauerstoffatome nicht unmittelbar miteinander gebunden sein dürfen, und/oder wobei ein oder mehrere H-Atome des Alkylrestes durch -F oder -Cl substituiert sein können; $R^4$ und $R^5$ können zusammen auch $-(CH_2)_4-$ oder $-(CH_2)_5-$ sein, wenn sie an ein Oxiran-, Dioxolan-, Tetrahydrofuran-, Tetrahydropyran-, Butyrolacton- oder Valerolacton-System gebunden sind;

$M^1$, $M^2$, $M^3$, $M^4$ sind gleich oder verschieden -O-, -S-, -CO-, -CO-O-, -O-CO-,-O-CO-O-, -CO-S-, -S-CO-, -CS-O-, -O-CS-, -S-CS-S-, -O-CS-O, -S-CO-S-, -CS-, $-CH_2-O-$, $-O-CH_2-$, $-CH_2-S-$, $-S-CH_2-$, -CH=CH-, -C≡C-, $-CH_2-CH_2-CO-O-$, $-O-CO-CH_2-CH_2-$ oder eine Einfachbindung;

$A^1$, $A^2$ sind gleich oder verschieden 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, wobei ein oder zwei H-Atome durch CN und/oder $CH_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F, Cl und/oder CN ersetzt sein kann, 1,3-Thiazol-2,5-diyl, wobei ein H-Atom durch F, Cl und/oder CN ersetzt sein kann, Thiophen-2,4-diyl, wobei ein H-Atom durch F, Cl und/oder CN ersetzt sein kann, Thiophen-2,5-diyl, wobei ein oder zwei H-Atome durch F, Cl und/oder CN ersetzt sein können, Piperazin-1,4-diyl, Piperazin-2,5-diyl, Naphthalin-2,6-diyl, wobei ein oder mehrere H-Atome durch F, Cl und/oder CN ersetzt sein können, Bicyclo[2.2.2]octan-1,4-diyl, wobei ein oder mehrere H-Atome durch F, Cl und/oder CN ersetzt sein können oder 1,3-Dioxaborinan-2,5-diyl;

n, m sind Null oder Eins, jedoch in der Summe maximal 1.

2. Fluorierte Phenanthren-Derivate der Formel (I) nach Anspruch 1, **dadurch gekennzeichnet, daß** $R^1$, $R^2$ gleich oder verschieden geradkettige oder verzweigte Alkylreste (mit oder ohne asymmetrisches C-Atom) mit 1 bis 16 C-Atomen sind, wobei auch eine oder mehrere $CH_2$-Gruppen durch -O-, Cyclopropan-1,2-diyl oder $-Si(CH_3)_2-$ ersetzt sein können, mit der Maßgabe, daß Sauerstoffe nicht unmittelbar verbunden sein dürfen, und/oder wobei ein oder mehrere H-Atome des Alkylrestes durch F substituiert sein können; $R^1$ oder $R^2$ können auch H sein, jedoch nicht beide gleichzeitig.

3. Fluorierte Phenanthrenderivate der Formel (I) nach Anspruch 2, **dadurch gekennzeichnet, daß** $R^1$, $R^2$ gleich oder verschieden geradkettige oder verzweigte Alkoxyreste mit 1 bis 10 C-Atomen sind, wobei auch eine, durch mindestens zwei weitere $CH_2$-Gruppen vom Kern getrennt, $CH_2$-Gruppe durch $-Si(CH_3)_2$ ersetzt sein kann.

4. Fluorierte Phenanthrenderivate der Formel (I) nach einem oder mehreren der Ansprüche 1 bis 3, **dadurch gekennzeichnet, daß** die Symbole $E^1$ und $E^4$ gleich oder verschieden -CF- oder -CH- und $E^2$, $E^3$, $E^5$ und $E^6$ -CH- bedeuten.

5. Fluorierte Phenanthrenderivate der Formel (I) nach Anspruch 4, **gekennzeichnet durch** die Formeln:

(Ia1) 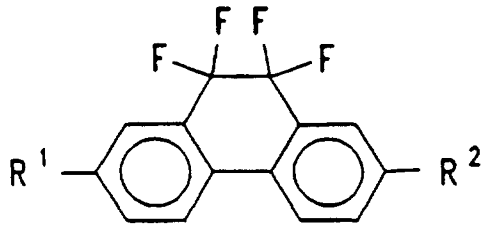

(Ia2) 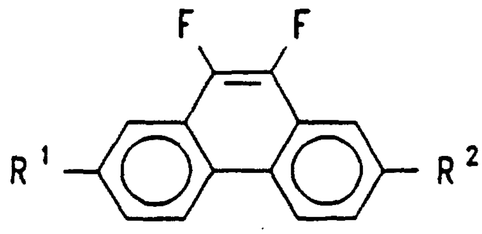

(Ia3) 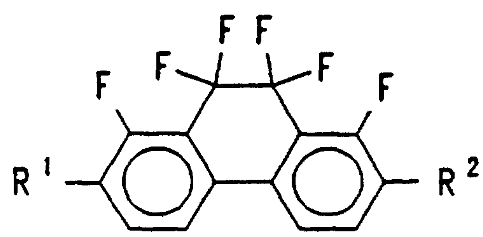

(Ia4) 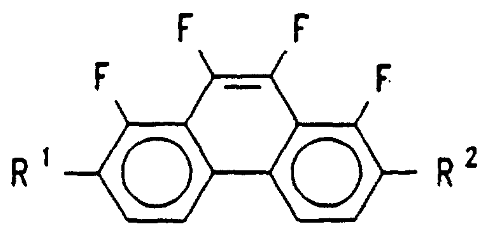

(Ia5) 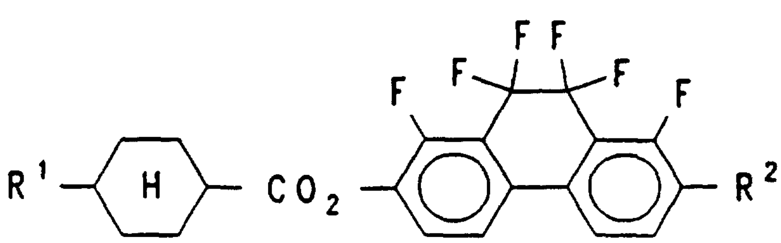

(Ia6)

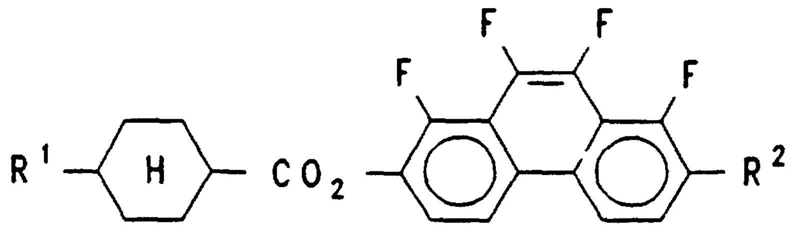

(Ia7)

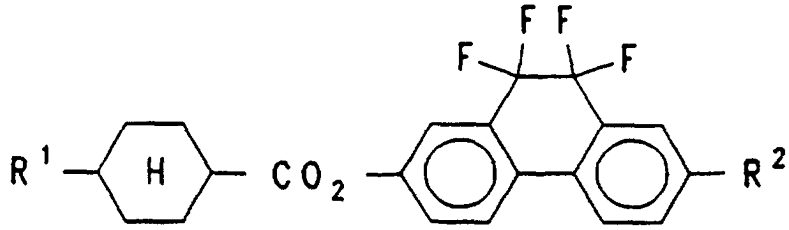

(Ia8)

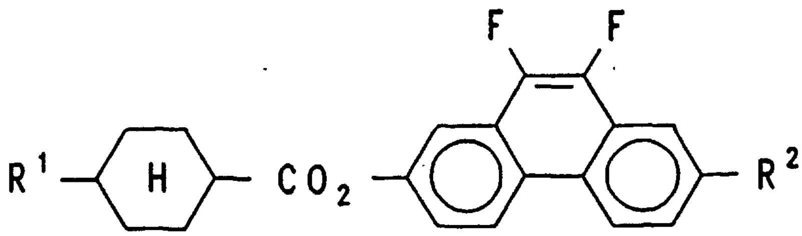

**6.** Verwendung von fluorierten Phenanthrenderivaten der Formel (I) nach einem oder mehreren der Ansprüche 1 bis 5 als Komponenten flüssigkristalliner Mischungen.

**7.** Flüssigkristallmischung, enthaltend eine oder mehrere Verbindungen der Formel (I) nach einem oder mehreren der Ansprüche 1 bis 5.

**8.** Flüssigkristallmischung nach Anspruch 7, **dadurch gekennzeichnet, daß** sie 0,1 bis 60 Gew.-% einer oder mehrerer Verbindungen der Formel (I) enthält.

**9.** Flüssigkristallmischung nach Anspruch 7 und/oder 8, **dadurch gekennzeichnet, daß** sie ferroelektrisch ist.

**10.** Schalt- und/oder Anzeigevorrichtung, enthaltend Trägerplatten, Elektroden, mindestens einen Polarisator, mindestens eine Orientierungsschicht sowie ein flüssigkristallines Medium, **dadurch gekennzeichnet, daß** das flüssigkristalline Medium eine Flüssigkristallmischung nach einem oder mehereren der Ansprüche 7 bis 9 ist.

## Claims

**1.** A fluorinated phenanthrene derivative of the formula (I)

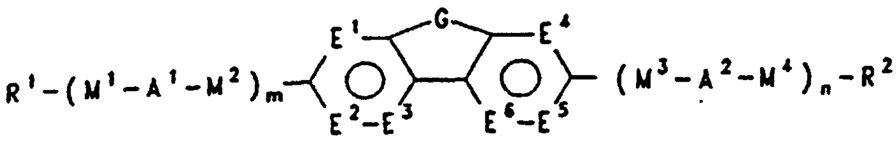 (1)

in which the symbols and indices have the following meanings:

$E^1$, $E^2$, $E^3$, $E^4$, $E^5$ and $E^6$ are identical or different and are -N-, -CF- or -CH-;

G is $-CF_2CF_2-$ or -CF=CF-;

$R^1$, $R^2$ are identical or different and are hydrogen, -CN, -F, -Cl, $-CF_3$, $-CHF_2$, $-CH_2F$, $-OCF_3$, $-OCHF_2$, $-OCH_2F$ or a straight-chain or branched alkyl radical having 1 to 20 carbon atoms (with or without an asymmetric carbon atom), where alternatively one or more $CH_2$ groups can be replaced by -O-, -S-, -CO-O, -O-CO-, -O-CO-, -O-CO-O-, -CO-, -CS-, -CH=CH-, -C≡C-, cyclopropane-1,2-diyl, $-Si(CH_3)_2-$, 1,4-phenylene, trans-1,4-cyclohexylene or trans-1,3-cyclopentylene, with the proviso that oxygen atoms and/or sulfur atoms must not be attached directly to one another and/or where one or more hydrogen atoms of the alkyl radical can be substituted by -F, -Cl, -Br, $-OR^3$, -SCN, -OCN or $-N_3$, or else are one of the following groups (optically active or racemic):

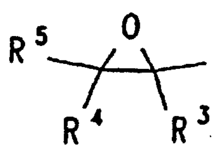
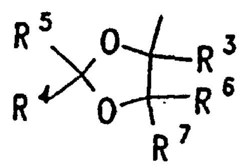
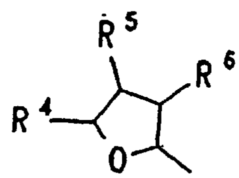

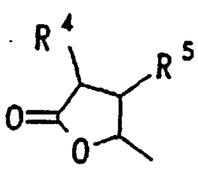
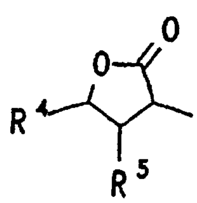
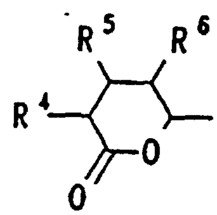

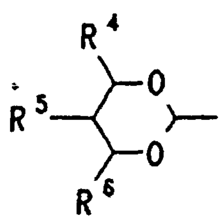
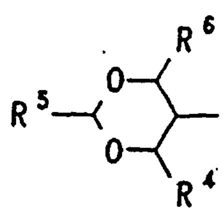
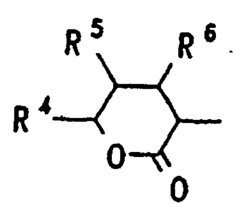

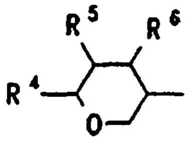 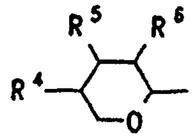 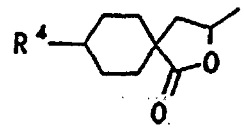

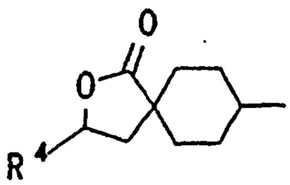 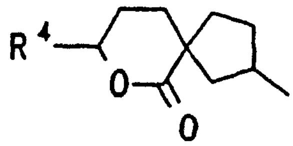 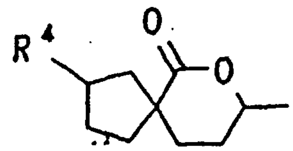

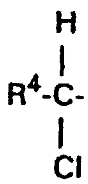 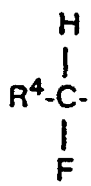 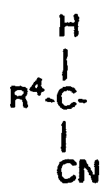 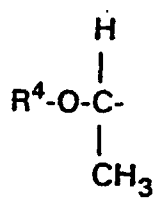 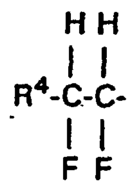

$R^3$, $R^4$, $R^5$, $R^6$, $R^7$ are identical or different and are hydrogen or a straight-chain or branched alkyl radical having 1-16 carbon atoms (with or without an asymmetric carbon atom), where alternatively one or more $CH_2$ groups can be replaced by -O- and/or -CH=CH-, with the proviso that oxygen atoms must not be attached directly to one another, and/or where one or more hydrogen atoms of the alkyl radical can be substituted by -F or -Cl; $R^4$ and $R^5$ can together also be $-(CH_2)_4-$ or $-(CH_2)_5-$ if they are attached to an oxirane, dioxolane, tetrahydrofuran, tetrahydropyran, butyrolactone or valerolactone system;

$M^1$, $M^2$, $M^3$, $M^4$ are identical or different and are -O-, -S-, -CO-, -CO-O-, -O-CO-, -O-CO-, -CO-S-, -S-CO-, -CS-O-, -O-CS, -S-CS-S-, -O-CS-O -S-CO-S-, -CS-, $-CH_2-O-$, $-O-CH_2-$, $-CH_2-S-$, $-S-CH_2$, -CH=CH-, -C≡C-, $-CH_2-CH_2-CO-O-$, $-O-CO-CH_2-CH_2-$ or a single bond;

$A^1$, $A^2$ are identical or different and are 1,4-phenylene, where one or more hydrogen atoms can be replaced by F, Cl and/or CN, pyrazine-2,5-diyl, where one or two hydrogen atoms can be replaced by F, Cl and/or CN, pyridazine-3,6-diyl, where one or two hydrogen atoms can be replaced by F, Cl and/or CN, pyridine-2,5-diyl, where one or more hydrogen atoms can be replaced by F, Cl and/or CN, pyrimidine-2,5-diyl, where one or two hydrogen atoms can be replaced by F, Cl and/or CN, trans-1,4-cyclohexylene, where one or two hydrogen atoms can be replaced by CN and/or $CH_3$, 1,3,4-thiadiazofe-2,5-diyl, 1,3-dioxane-2,5-diyl, 1,3-dithiane-2,5-diyl, 1,3-thiazole-2,4-diyl, where one hydrogen atom can be replaced by F, Cl and/or CN, 1,3-thiazole-2,5-diyl where one hydrogen atom can be replaced by F, Cl and/or CN, thiophene-2,4-diyl, where one hydrogen atom can be replaced by F, Cl and/or CN, thiophene-2,5-diyl where one or two hyrogen atoms can be replaced by F, Cl and/or CN, piperazine-1,4-diyl, piperazine-2,5-djyl. naphthalene-2,6-diyl, where one or more hydrogen atoms can be replaced by F, Cl and/or CN, bicyclo[2.2.2]octane-1,4-diyl, where one or more hydrogen atoms can be replaced by F, Cl and/or CN, or 1,3-dioxaborinane-2,5-diyl;

n, m are zero or one, but in total not more than 1.

2. A fluorinated phenanthrene derivative of formula (I) as claimed in claim 1, wherein $R^1$, $R^2$ are identical or different and are straight-chain or branched alkyl radicals (with or without an asymmetric carbon atom) having 1 to 16 carbon atoms, where alternatively one or more $CH_2$ groups can be replaced by -O-, cyclopropane-1,2-diyl or $-Si(CH_3)_2-$, with the proviso that oxygens must not be directly connected, and/or where one or more hydrogen atoms of the alkyl radical can be substituted by F; $R^1$ or $R^2$ can also be H, but not both simultaneously.

3. A fluorinated phenanthrene derivative of the formula (I) as claimed in claim 2, wherein $R^1$, $R^2$ are identical or different and are straight-chain or branched alkoxy radicals having 1 to 10 carbon atoms, where alternatively one $CH_2$ group, separated by at least two further $CH_2$ groups from the nucleus, can be replaced by $-Si(CH_3)_2-$.

4. A fluorinated phenanthrene derivative of formula (I) as claimed in one or more of claims 1 to 3, wherein the symbols $E^1$ and $E^4$ are identical or different and are -CF- or -CH- and $E^2$, $E^3$, $E^5$ and $E^6$ are -CH-.

5. A fluorinated phenanthrene derivative of formula (I) as claimed in claim 4, which has the formula:

(Ia1) 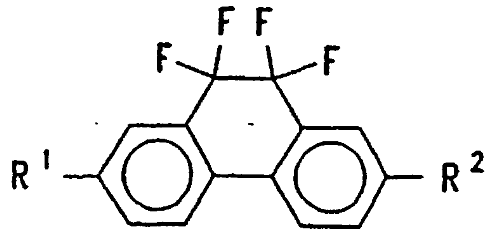

(Ia2) 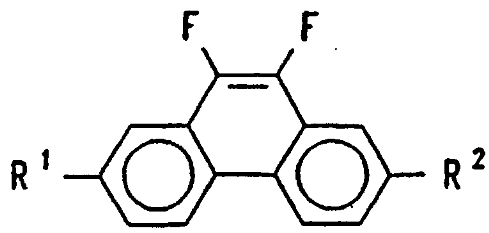

(Ia3) 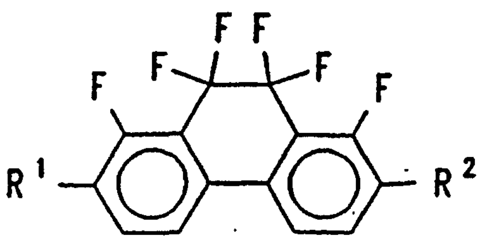

(Ia4) 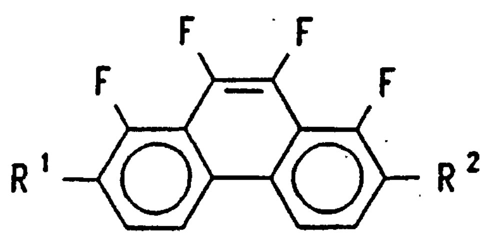

(Ia5) 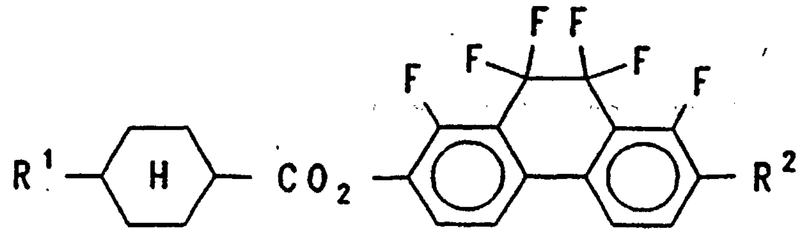

(Ia6) 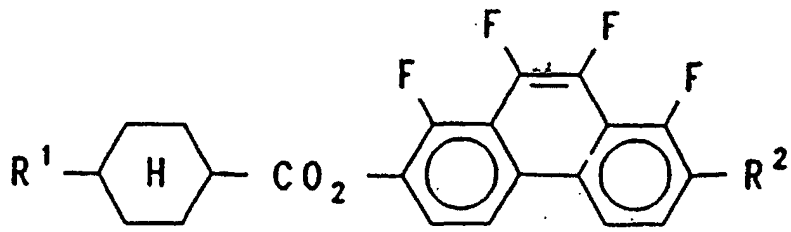

(Ia7) 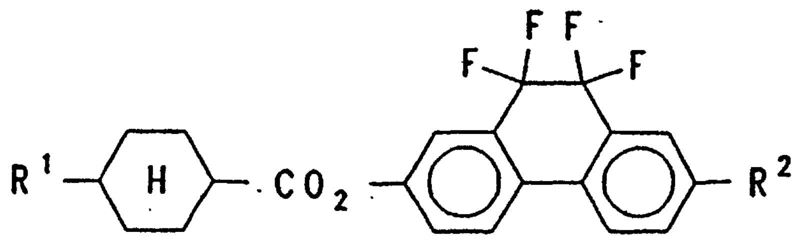

(Ia8) 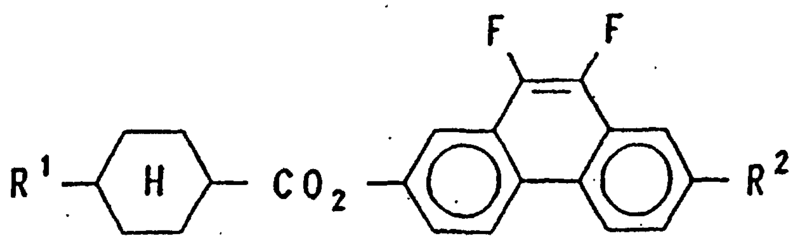

**6.** The use of a fluorinated phenanthrene derivative of the formula (I) as claimed in one or more of claims 1 to 5 as a component of liquid-crystalline mixtures.

**7.** A liquid-crystal mixture comprising one or more compounds of the formula (I) as claimed in one or more of claims 1 to 5.

**8.** A liquid-crystal mixture as claimed in claim 7, which contains from 0.1 to 60 % by weight of one or more compounds of the formula (I).

**9.** A liquid-crystal mixture as claimed in claim 7 or 8, which is ferroelectric.

**10.** A switching and/or display device comprising carrier plates, electrodes, at least one polarizer, at least one alignment layer and a liquidcrystalline medium, wherein the liquid-crystalline medium is a liquid-crystal mixture as claimed in one or more of claims 7 to 9.

## Revendications

1. Dérivés fluorés de la phénanthrène de formule (I)

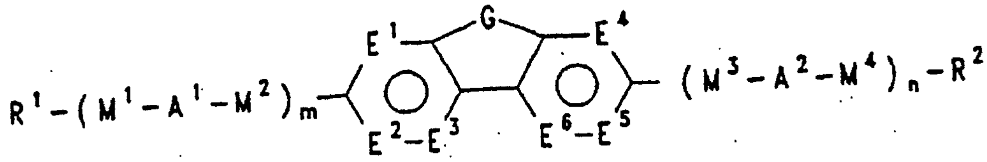

(I)

où les symboles et les indices possèdent les significations suivantes :

| | |
|---|---|
| $E^1$, $E^2$, $E^3$, $E^4$, $E^5$, $E^6$, | identiques ou différents, représentent des groupes -N-, -CF- ou -CH- ; |
| G | représente des groupes $-CF_2CF_2-$ ou -CF=CF- ; |
| $R^1$, $R^2$, | identiques ou différents, représentent des atomes d'hydrogène, des groupes -CN, -F, -Cl, $-CF_3$, $-CHF_2$, $-CH_2F$, $-OCF_3$, $-OCHF_2$, $-OCH_2F$ ou un reste alkyle linéire ou ramifié possédant 1 à 20 atomes de carbone (avec ou sans atome de carbone asymétrique), un ou plusieurs groupes $CH_2$ pouvant être remplacés par des groupes -O-, -S-, -CO-O-, -O-CO-, -O-CO-O-, -CO-, -CS-, -CH=CH-, -C≡C-, cyclopropane-1,2-diyle, $-Si(CH_3)_2-$, 1,4-phénylène, trans-1,4-cyclohexylène ou trans-1,3-cyclopentylène, à condition que des atomes d'oxygène et/ou atomes de soufre ne puissent pas être liés les uns aux autres directement, et/ou un ou plusieurs atomes de H du reste alkyle pouvant être remplacés par des groupes -F, -Cl, -Br, $-OR^3$, -SCN, -OCN ou $-N_3$, ou aussi un ou plusieurs des groupes suivants (optiquement actifs ou racémiques) |

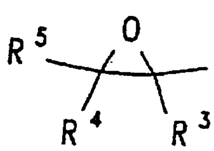
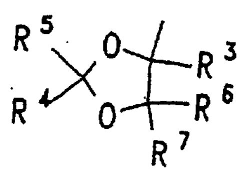
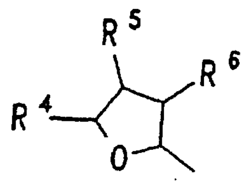

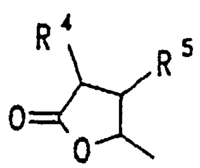
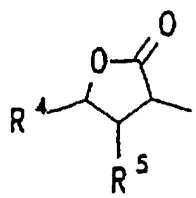
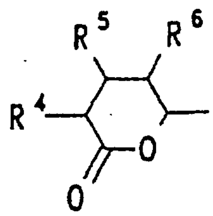

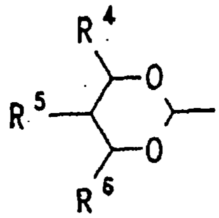
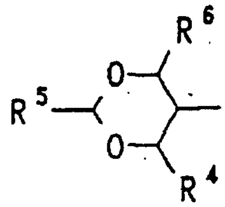
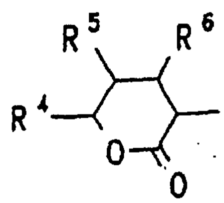

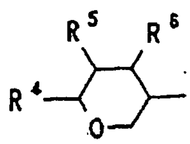 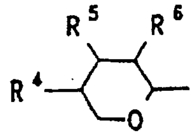 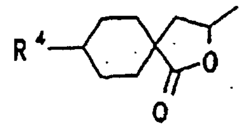

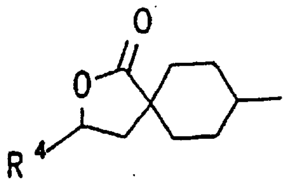 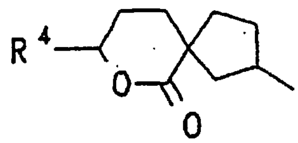 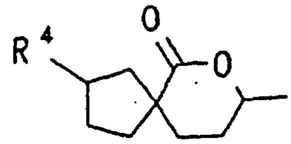

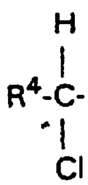 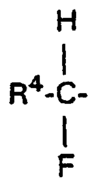 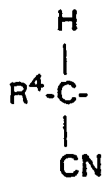 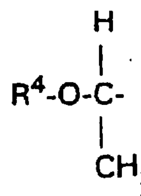 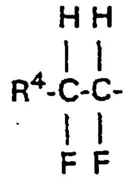

| | |
|---|---|
| $R^3$, $R^4$, $R^5$, $R^6$, $R^7$, | identiques ou différents, représentent un atome d'hydrogène ou un reste alkyle linéaire ou ramifié présentant 1 à 16 atomes de carbone (avec ou sans atome de carbone asymétrique), un ou plusieurs groupes $CH_2$ pouvant être remplacés par des groupes -O- et/ou -CH=CH-, à condition que des atomes d'oxygène ne puissent pas être liés directement les uns aux autres et/ou un ou plusieurs atomes de H du reste alkyle pouvant être substitués par des substituants -F ou -Cl ; $R^4$ et $R^5$ peuvent représenter ensemble un groupe $-(CH_2)_4-$ ou $-(CH_2)_5-$, lorsqu'ils sont liés à un système oxiranne, dioxolanne, tétrahydrofuranne, tétrahydropyrane, butyrolactone ou valérolactone ; |
| $M^1$, $M^2$, $M^3$, $M^4$ | sont identiques ou différents et représentent -O-, -S-, -CO-, -CO-O-, -O-CO-, -O-CO-O-, -CO-S-, -S-CO-, -CS-O-, -O-CS-, -S-CS-S-, -O-CS-O-, -S-CO-S-, -CS-, $-CH_2O-$, $-OCH_2-$, $-CH_2S-$, $-S-CH_2-$, -CH=CH-, -C≡C-, $-CH_2-CH_2-CO-O-$, $-O-CO-CH_2-CH_2-$ ou une simple liaison, |
| $A^1$, $A^2$ | sont identiques ou différents et représentent un groupe 1,4-phénylène; un ou plusieurs atomes de H pouvant être remplacés par des groupes F, Cl et/ou CN, pyrazine-2,5-diyle, un ou deux atomes de H pouvant être remplacés par des groupes F, Cl et/ou CN, pyridazine-3,6-diyle, un ou deux atomes de H pouvant être remplacés par des groupes F, Cl et/ou CN, pyridine-2,5-diyle, un ou plusieurs atomes de H pouvant être remplacés par des groupes F, Cl et/ou CN, pyrimidine-2,5-diyle, un ou deux atomes de H pouvant être remplacés par des groupes F, Cl et/ou CN, trans-1,4-cyclohexylène, un ou deux atomes de H pouvant être remplacés par des groupes CN et/ou $CH_3$, (1,3,4)-thiadiazol-2,5-diyle, 1,3-dioxanne-2,5-diyle, 1,3-dithiane-2,5-diyle, 1,3-thiazol-2,4-diyle, un atome de H pouvant être remplacé par des groupes F, Cl et/ou CN, 1,3-thiazol-2,5-diyle, un atome de H pouvant être remplacé par des groupes F, Cl et/ou CN, thiophène-2,4-diyle, un atome de H pouvant être remplacé par des groupes F, Cl et/ou CN, thiophène-2,5-diyle, un ou deux atomes de H pouvant être remplacés par des groupes F, Cl et/ou CN, pipérazine-1,4-diyle, pipérazine-2,5-diyle, naphtalène-2,6-diyle, un ou plusieurs atomes de H pouvant être remplacés par des groupes F, Cl et/ou CN, bicyclo[2.2.2]-octane-1,4-diyle, un ou plusieurs atomes de H pouvant être remplacés par des groupes F, Cl et/ou CN ou 1,3-dioxaborinane-2,5-diyle; |
| n, m | valant zéro ou un, toutefois la somme doit être égal à 1 au maximum. |

**2.** Dérivés fluorés de la phénanthrène de formule (I) selon la revendication 1, **caractérisés en ce que** $R^1$, $R^2$, identiques ou différents, représentent des restes alkyle linéaires ou ramifiés (avec ou sans atome de carbone asymétrique) présentant 1 à 16 atomes de carbone, un ou plusieurs groupes $CH_2$ pouvant être remplacés par par des groupes -O-, cyclopropane-1,2-diyle ou $-Si(CH_3)_2-$, à condition que des atomes d'oxygène ne puissent pas être liés directement les uns aux autres, et/ou un ou plusieurs atomes de H du reste alkyle pouvant être substitués par un substituant F ; $R^1$ ou $R^2$ peuvent être également H, mais pas les deux simultanément.

**3.** Dérivés fluorés de la phénanthrène de formule (I) selon la revendication 2, **caractérisés en ce que** $R^1$, $R^2$ sont identiques ou différents et représentent des restes alkoxy linéaires ou ramifiés présentant 1 à 10 atomes de carbone, un groupe $CH_2$, séparé du noyau par au moins deux autres groupes $CH_2$, pouvant être remplacé par un groupe $-Si(CH_3)_2$.

**4.** Dérivés fluorés de la phénanthrène de formule (I) selon une ou plusieurs revendications 1 à 3, **caractérisés en ce que** les symboles $E^1$ et $E^4$ sont identiques ou différents et représentent des groupes -CF- ou -CH- et $E^2$, $E^3$, $E^5$ et $E^6$ représentent un groupe -CH-.

**5.** Dérivés fluorés de la phénanthrène de formule (I) selon la revendication 4, **caractérisés par** les formules :

(Ia1) 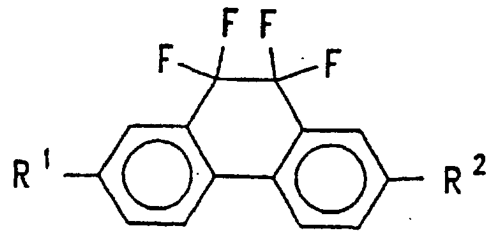

(Ia2) 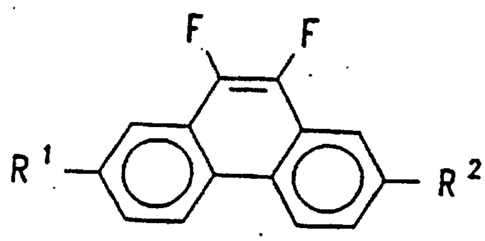

(Ia3) 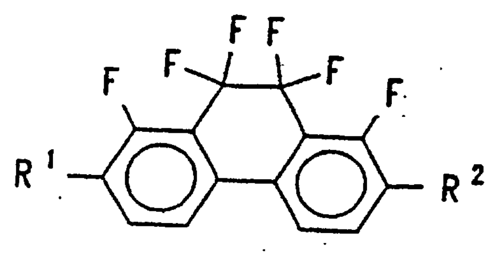

(Ia4) 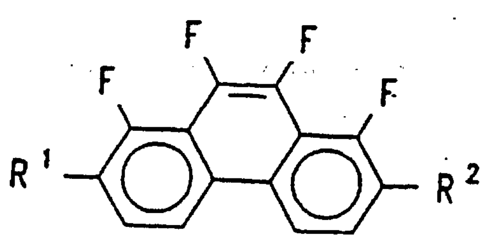

(Ia5) 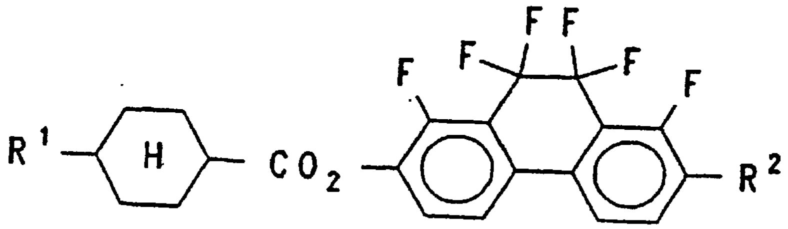

(Ia6) 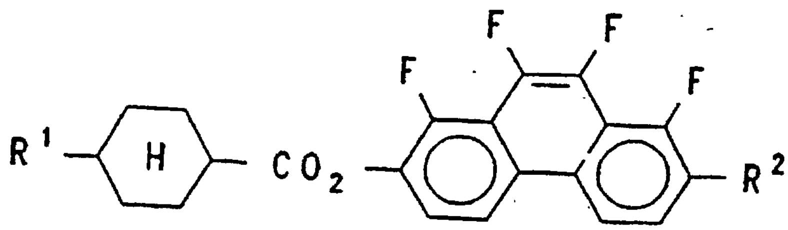

(Ia7) 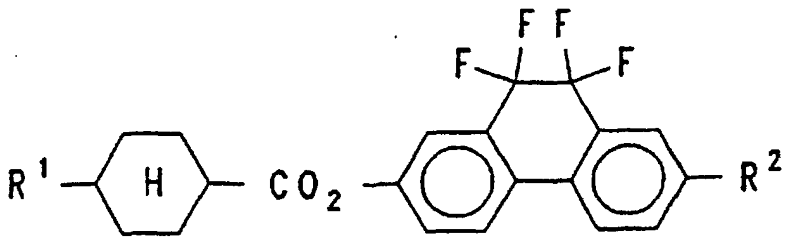

(Ia8) 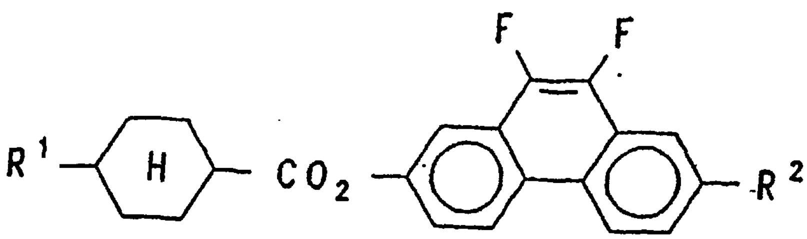

**6.** Utilisation de dérivés fluorés de la phénanthrène de formule (I) selon une ou plusieurs des revendications 1 à 5 en tant que constituant de mélanges de cristaux liquides.

**7.** Mélange de cristaux liquides contenant un ou plusieurs composés de formule (I) selon une ou plusieurs des revendications 1 à 5.

**8.** Mélange de cristaux liquides selon la revendication 7, **caractérisé en ce qu'**il présente une teneur de 0,1 à 60 % en poids en un ou plusieurs composés de formule (I).

**9.** Mélange de cristaux liquides selon la revendication 7 et/ou 8, **caractérisé en ce qu'**il est ferroélectrique.

**10.** Dispositif de commutation et/ou d'affichage présentant des plaques supports, des électrodes, au moins un polariseur, au moins une couche d'orientation , ainsi qu'un milieu de cristaux liquides, **caractérisé en ce que** le milieu de cristaux liquides est un mélange de cristaux liquides selon une ou plusieurs des revendications 7 à 9.